# EUROPEAN PATENT APPLICATION

(11) **EP 2 106 822 A1**
(43) Date of publication of application: **07.10.2009**
(21) Application number: 07860087.1
(22) Date of filing: 25.12.2007
(51) Int. Cl.: A61N 5/06

(54) **OPTICAL HAIR GROWTH CONTROL DEVICE**

(30) Priority: 25.12.2006 JP 2006348635; 25.12.2006 JP 2006348636; 25.12.2006 JP 2006347630
(71) Applicant: Panasonic Electric Works Co., Ltd, Kadoma-shi Osaka 571-8686 (JP)
(72) Inventor: HAMADA, Chosei, Kadoma-shi, Osaka (JP); KINOSHITA, Masato, Kadoma-shi, Osaka (JP); SATOH, Yasuhiro, Kobe-shi Hyogo, 658-0082 (JP); NOGITA, Toshitatsu, Tokyo 177-0041 (JP)
(74) Representative: Appelt, Christian W.
(86) International application number: PCT/JP2007/074858
(87) International publication number: WO 2008/078750

(57) **Abstract**

An optical hair growth modulation device modulates hair growth by irradiating modulating light to skin. This device is provided with a modulating light irradiator for irradiating modulating light, a measuring light irradiator for irradiating measuring light for measurement of a target site of the human body to the target site, a light receiver for receiving the measuring light reflected from the target site, a processor for processing information of the measuring light received by the light receiver, and an irradiation determinator for judging whether or not to make the light irradiation from the modulating light irradiator based on a processing result of the processor. The irradiation determinator is configured to control irradiation of modulating light from the modulating light irradiator based on the judgment thereof. This device is able to judge whether or not a target site is suitable as a site to be irradiated with modulating light by using measuring light, thereby preventing an unsuitable site such as an eye ball from being accidentally irradiated with the modulating light.

## Description

### TECHNICAL FIELD

The present invention relates to an optical hair growth modulation device for promoting or inhibiting hair growth by irradiating light.

### BACKGROUND ART

Hair (including body hair and scalp hair) is known to have a hair cycle during which hair changes in cycles consisting of a growth period, a regressive period and a rest period. When light, having a quantity of light or luminous energy that does not cause changes in cell morphology as observed with existing therapeutic lasers, is irradiated during the above-mentioned rest period, growth of hair during the growth period of the hair cycle has been confirmed to proceed rapidly. In this case, the absence of the occurrence of cell damage as well as the absence of the occurrence of adverse side effects such as burns has also been confirmed. In addition, when hair is irradiated with light during the growth period of the hair cycle, hair growth has been confirmed to be effectively inhibited. Furthermore, although the reason why hair growth is promoted or inhibited when hair is irradiated with light at a level that does not cause changes in cell morphology during the rest period or growth period is not clear, based on the results of analyses at the RNA level, activation of inflammatory cytokines is thought to occur as a result of being irradiated with light, and the resulting promotion or inhibition of hair growth is thought to be the result of this activation of inflammatory cytokines.

However, in promoting or inhibiting hair growth by irradiating light for modulating hair growth as described above to human skin, it is required to only radiate light at a target site on the body to be irradiated with that light. However, sites on the body to be irradiated differ among individuals resulting in light being irradiated at sites other than the target site to be irradiated. Since there are sites on the body surface where excessive irradiation of light is not desirable, it is necessary to confirm the site to be irradiated in advance. Since irradiating the eyes with light is particularly dangerous, it is important to avoid exposing the eyes to such light.

Japanese Patent Application Laid-open No. 2002-177405 proposes a device for preventing the eye balls from being irradiated with light. This device uses a sensor that detects contact with skin, and is composed so that light is only irradiated when the sensor is in contact with skin. However, in this type of device, it is necessary that the sensor be in continuous contact with skin, thereby resulting in the problem of poor ease of use.

### DISCLOSURE OF THE INVENTION

With the foregoing in view, an object of the present invention is to provide an optical hair growth modulation device capable of safely irradiating light.

The optical hair growth modulation device as claimed in the present invention is provided with a modulating light irradiator for irradiating modulating light for modulation of hair growth to a target site of a human body, a measuring light irradiator for irradiating measuring light for measurement of the human body to the target site of the human body, a light receiver for receiving the measuring light reflected from the target site, a processor for processing information of the measuring light received by the light receiver, and an irradiation determinator for judging whether or not to irradiate the modulating light from the modulating light irradiator based on a processing result of the processor; wherein, the irradiation determinator is configured to control irradiation of modulating light from the modulating light irradiator based on the judgment thereof. In this manner, the device of the present invention is able to judge whether or not a target site is suitable as a site to be irradiated with modulating light by using measuring light irradiated towards a target site. The device can therefore be used safely without accidentally irradiating an unsuitable site such as an eye ball with the modulating light.

The above-mentioned processor can be configured to output an estimated shape of the target site. In this case, since the irradiation determinator is configured to judge not to irradiate the modulating light from the modulating light irradiator in the case the estimated shape coincides with the shape of a human eye ball, irradiation of the eye ball with the modulating light is prevented.

In addition, the irradiation determinator can also be configured to judge not to irradiate the modulating light from the modulating light irradiator in the case the estimated shape has a regularly repeated pattern of a convexo-concave shape. As a result, skin lesions can be recognized, thereby making it possible to exclude this site from the target site to be irradiated with the modulating light.

Moreover, the processor can be configured to obtain curvature of a cross-section of the target site by means of a light-section method and output the curvature. In this case, the irradiation determinator is configured to judge not to irradiate the modulating light from the modulating light irradiator in the case the curvature coincides with the curvature inherent to the human eye ball, thereby making it possible to prevent the eye ball from being irradiated with the modulating light.

In addition, the measuring light can be light polarized in a particular direction, and the light receiver can be configured to obtain polarization information of this measuring light. The shape of a target site can be accurately determined from this polarization information, enabling more suitable control.

Furthermore, the processor can also be configured to calculate the amount of the measuring light received by the light receiver. In this case, the irradiation determinator is configured to judge whether or not the modulating light is to be irradiated from the modulating light irradiator based on the amount of the measuring light.

The measuring light has preferably a wavelength in a range of 200 nm to 400 nm.

Moreover, the light receiver can be configured such that a plurality of light receiving elements are arranged in a two-dimensional array, and the irradiation determinator can be configured to judge not to irradiate the modulating light from the modulating light irradiator when the amount of light received by a predetermined number of light receiving elements or more is equal to or less than a prescribed value. As a result of employing this configuration, dangerous sites can be detected over a wide range, thereby making it possible to improve the safety of the device.

Polarized measuring light can also be used in the case of calculating the amount of measuring light at the light receiver.

Moreover, in the present invention, in order to determine a site able to be irradiated with modulating light, the measuring light irradiator can be configured to irradiate three types of red, green and blue (RGB) measuring light, and the light receiver can be configured to measure the amount of light of each red, green and blue component of the measuring light.

### BRIEF DESCRIPTION OF THE DRAWINGS

FIG. 1 is a schematic drawing showing the configuration of an optical hair growth modulation device as claimed in a first embodiment of the present invention;
FIG. 2 is a flow chart for explaining the operation of the above-mentioned device;
FIG. 3 is an explanatory drawing showing a mask used in the above-mentioned device;
FIG. 4 is an explanatory drawing illustrating a technique for assessing an eye ball during image processing with the above-mentioned device;
FIG. 5 is a flow chart indicating the above-mentioned technique for assessing an eye ball;
FIG. 6 is an explanatory drawing illustrating a technique for assessing skin lesions during image processing with the above-mentioned device;
FIG. 7 is a flow chart indicating the above-mentioned technique for assessing skin lesions;
FIG. 8 is a schematic drawing showing an example of a measuring head in the above-mentioned device;
FIG. 9 is an explanatory drawing illustrating a technique for assessing a mole with the above-mentioned device;
FIG. 10 is a flow chart indicating the above-mentioned technique for assessing a mole;
FIG. 11 is a schematic drawing showing the configuration of an optical hair growth modulation device as claimed in a second embodiment of the present invention;
FIG. 12 is a flow chart for explaining the operation of the above-mentioned device;
FIG. 13 is a schematic drawing showing the configuration of an optical hair growth modulation device as claimed in a third embodiment of the present invention;
FIG. 14 is a flow chart for explaining the operation of the above-mentioned device;
FIG. 15 is a schematic drawing showing the configuration of an optical hair growth modulation device as claimed in a fourth embodiment of the present invention;

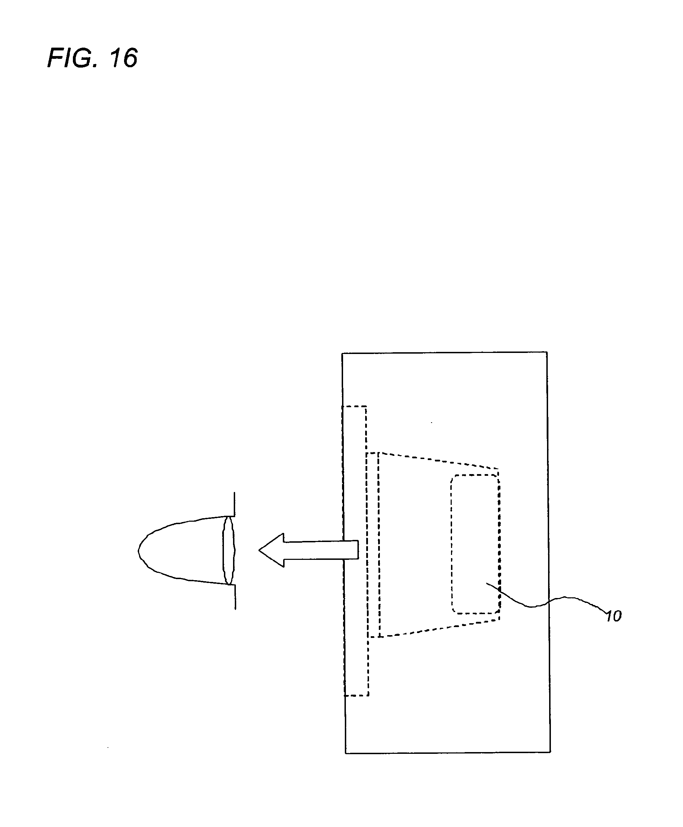

FIG. 16 is a schematic drawing showing the configuration of an optical hair growth modulation device as claimed in a fifth embodiment of the present invention;
FIG. 17 is a drawing for explaining the present invention that shows the results of measuring the spectral reflectance of skin at various wavelengths of light using samples from three persons; and
FIG. 18 is a drawing for explaining the present invention that illustrates the relationship between light wavelength and the absorbance of melanin alone.

### BEST MODE FOR CARRYING OUT THE INVENTION

### (First Embodiment)

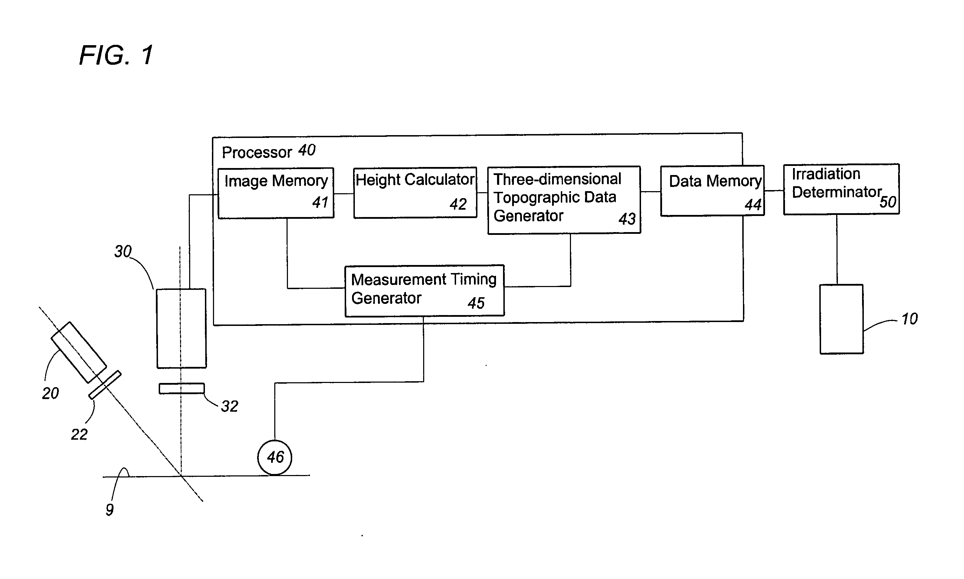

FIG. 1 shows an optical hair growth modulation device as claimed in a first embodiment of the present invention. This device is provided with a modulating light irradiator 10 that irradiates modulating light for modulation of hair growth to a target site of a human body. The modulating light irradiator 10 is provided with a light source in the form of a xenon flash lamp, and outputs modulating light in the form of flashing light having a wavelength of, for example, 400 to 600 nm through a suitable filter. Consequently, the modulating light irradiator 10 is provided with a drive circuit and a filter for emitting modulating light for a short time of 1 ms or less by driving the xenon flash lamp with a one-shot pulse.

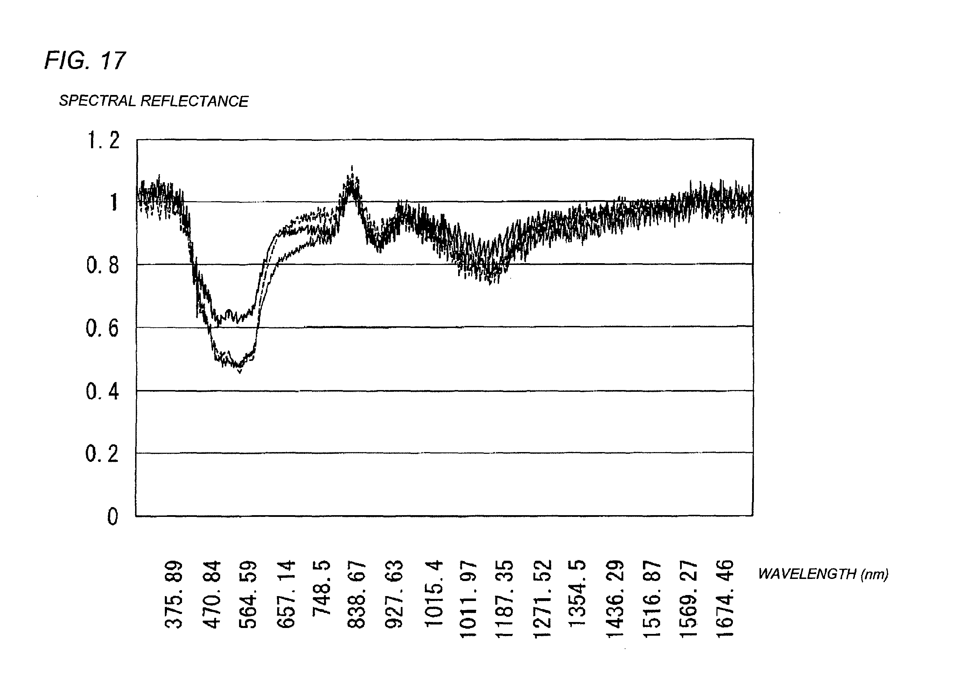

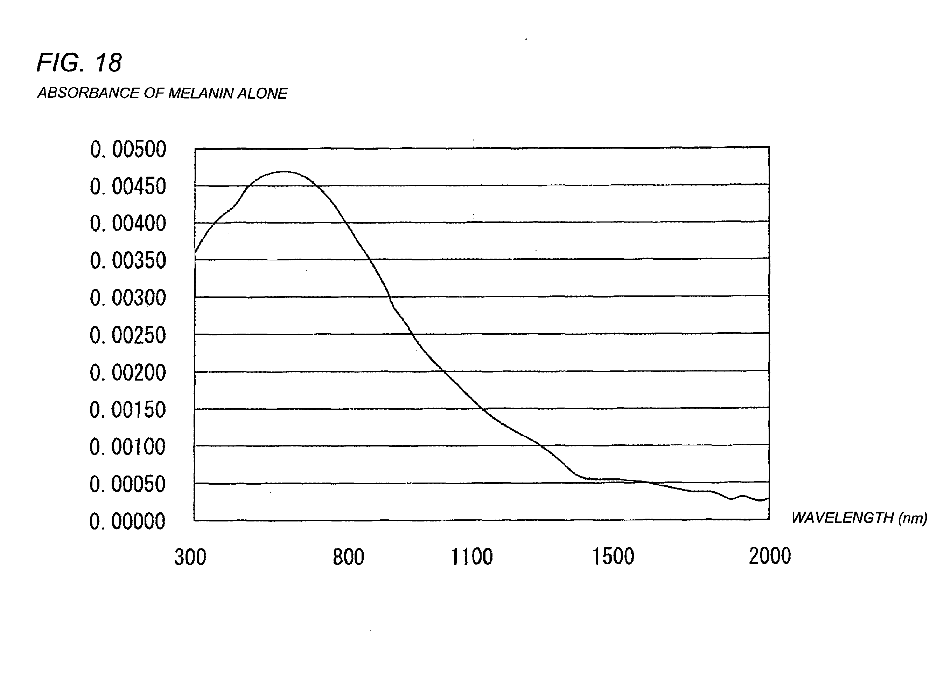

Modulating light irradiated from the modulating light irradiator 10 is required to be housed in the skin, and when selecting the wavelength thereof, spectral reflectance of the skin was measured at each wavelength of light using samples from three Japanese persons as shown in FIG. 17. Since spectral reflectance in the vicinity of 400 to 600 nm decreased for all samples, light in this wavelength range was determined to have the property of being easily absorbed. This is due to the considerable effects of melanin present in skin. FIG. 18 indicates the spectral properties of the absorbance of melanin alone. Melanin alone absorbs 39% of irradiated light in the case of irradiated light having a wavelength of 567 nm. On the basis thereof, the use of light having a wavelength within the range of 400 to 600 nm that is easily absorbed by the skin for the modulating light make it possible to stimulate hair with light at a low output. Furthermore, ultraviolet light having a wavelength of 400 nm or less that is harmful to the skin is cut out with a UV cutoff filter. Furthermore, the modulating light used in the present invention is not necessarily limited to the wavelength range described above, but rather modulating light can be used having a wavelength range of, for example, 400 to 1000 nm.

Modulating light irradiated from the xenon flash lamp is irradiated at illuminance of 1,500,000 to 7,000,000 lux and a flash duration (half value of peak power) of 100 to 700 µs. Since the quantity of irradiated light can be determined as the product of illuminance and flash duration, this becomes 150 to 4900 lux·s. In addition, irradiation energy (J/cm²) is the product of irradiation power (W) and irradiation time (seconds), and by controlling the irradiation power (W) and irradiation time (seconds) so that the irradiation energy K is, for example, 0.1 J/cm², the occurrence of adverse side effects attributable to irradiation can be reliably suppressed. Accompanying this, when comparing the case of irradiating light once/day at intervals of several days and the case of repeatedly irradiating light once/day for about 5 to 10 consecutive days, the latter allows the use of a lower power light source.

Hair (including body hair and scalp hair) is known to have a hair cycle during which hair changes in cycles consisting of a growth period, a regressive period and a rest period. The inventors of the present invention confirmed in experiments using mice that if light is irradiated to the skin under the above irradiation conditions, hair growth is effectively inhibited, without causing changes in cell morphology as is observed with existing therapeutic lasers and the like and without causing destruction of cells, if the light is irradiated during the growth period of the hair cycle. Furthermore, hair growth during the growth period of the hair cycle has been confirmed to proceed rapidly if light is irradiated during the rest period of the hair cycle. In addition, adverse side effects such as burns have also been confirmed to not occur.

Although the reason why hair growth is inhibited when hair is irradiated with light at a level that does not cause changes in cell morphology during the growth period is not clear, based on the results of analyses at the RNA level, activation of inflammatory cytokines is thought to occur as a result of being irradiated with light, and the resulting inhibition of hair growth is thought to be the result of this activation of inflammatory cytokines.

Furthermore, irradiation power was changed corresponding to the rate of change of spectral reflectance of the skin an irradiated target site based on the spectral reflectance at 500 to 600 nm at which effects occurred that caused changes in the hair cycle. When the reflectance of a specific wavelength serving as a reference is defined as R0, the reflectance of the irradiated skin is defined as R1, and the power of the light source for which activation of inflammatory cytokines was achieved for the skin of R0 is defined as P0, then the irradiation power P can be determined with the formula P = R1/R0 × P0. Since the energy having an effect on the hair cycle is a constant value, in the case spectral reflectance of the skin is high, power should be increased and irradiation time should be shortened.

Furthermore, since the duration of the growth period, regressive period and rest period of the hair cycle varies according to the location such as the scalp, light is irradiated after first determining whether or not the hair cycle is in the growth period for the location where hair growth is desired to be inhibited.

As shown in FIG. 1, the device of the present invention is provided with a measuring light irradiator 20 that irradiates measuring light for verifying a target site where modulating light is to be irradiated, and a light receiver 30 that receives measuring light reflected from the target site. Although the device of the present invention is basically configured to be used without making contact with the skin, it can also be used in contact with the skin.

The measuring light irradiator 20 contains only a line light source and a drive circuit thereof, and is configured so that, for example, line light having a wavelength of 200 to 400 nm is used as measuring light, and that light is output towards a skin surface 9 of a human body at an angle of 45°.

The light receiver 30 is composed of a CCD camera, and the optical axis thereof is arranged so as to be perpendicular to the skin surface 9 at an angle of 45° to the optical axis of the measuring light irradiator 2, and converts measuring light reflected from the skin surface to an electric signal. Since the measuring light irradiator 20 outputs line light at an angle relative to the skin surface 9, surface irregularities in the skin surface can be identified by reflected light received by the light receiver 30. Moreover, since the measuring light irradiator 20 and the light receiver 30 move along the skin surface, three-dimensional topographic data of the skin surface can be obtained.

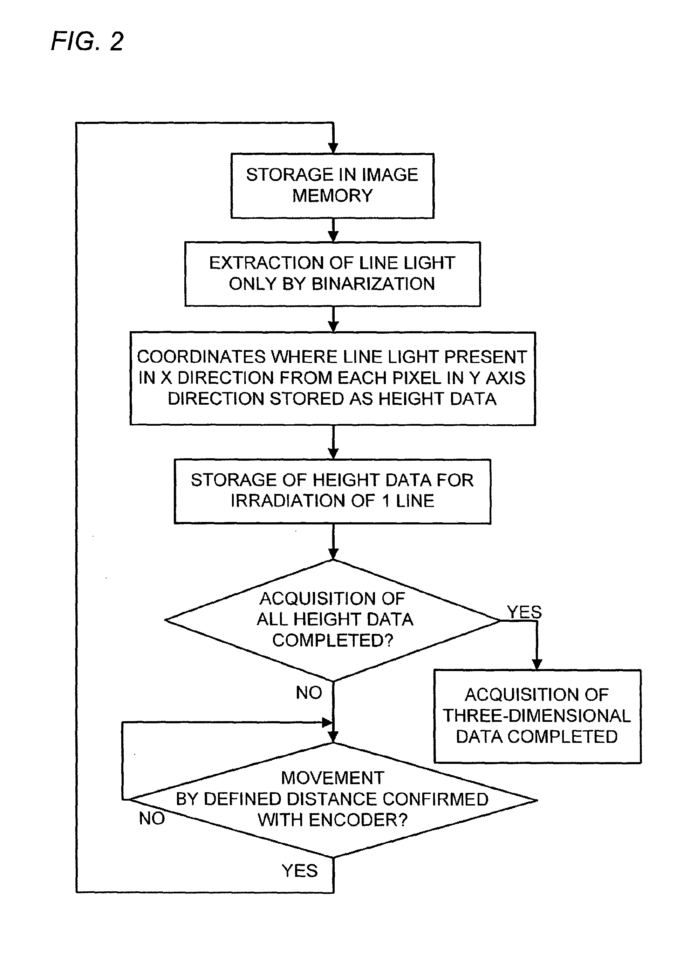

This device is also provided with a processor 40 that processes electric signals obtained from the light receiver 30. This processor 40 is composed of an image memory 41 for generating images of the skin surface from the output of the light receiver 30 and storing those images, a height calculator 42 for calculating the height of each part of the skin surface from the images, a three-dimensional topographic data generator 43 for generating three-dimensional topographic data of the skin surface, a data memory 44 for storing the three-dimensional topographic data, and a measurement timing generator 45 for determining measurement timing corresponding to an output from an encoder 46 for measuring locations irradiated with the measuring light. The processor 40 is configured to obtain three-dimensional topographic data of a target skin surface by carrying out the operation indicated in the flow chart of FIG. 2.

However, in the case measuring light is irradiated to the skin surface 9 and the reflected light thereof is measured, reflected light that has reflected at the skin surface 9 and reflected light that has passed through the skin are both present. However, although reflected light that has reflected at the skin surface 9 is polarized light in the same manner as the measuring light, light that has passed through the skin is affected by changes in the angle of polarization caused by internal tissue. Consequently, in the present embodiment, by using light polarized in a certain direction by a polarizing filter 22 for the measuring light, and installing a polarizing filter 32 having the same polarization direction as the measuring light in front of the light receiver 30, only measuring light reflected at the skin surface 9 is allowed to enter the light receiver 30, and as a result thereof, the three-dimensional shape of the skin surface 9 can be measured accurately.

A judgment is made as to whether or not modulating light is to be irradiated to a target site from the modulating light irradiator 1 based on whether or not the three-dimensional topographic data obtained in the manner described above coincides with a predetermined shape in an irradiation determinator 50, and in the case a result is obtained such that irradiation is permitted, the modulating light irradiator 10 is operated and modulating light is irradiated to the target site.

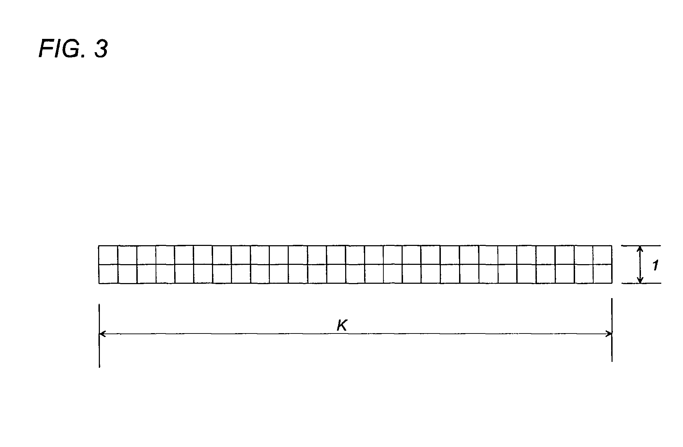

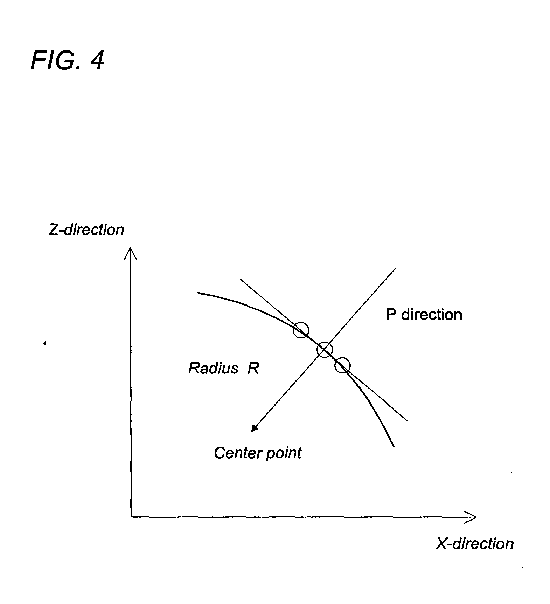

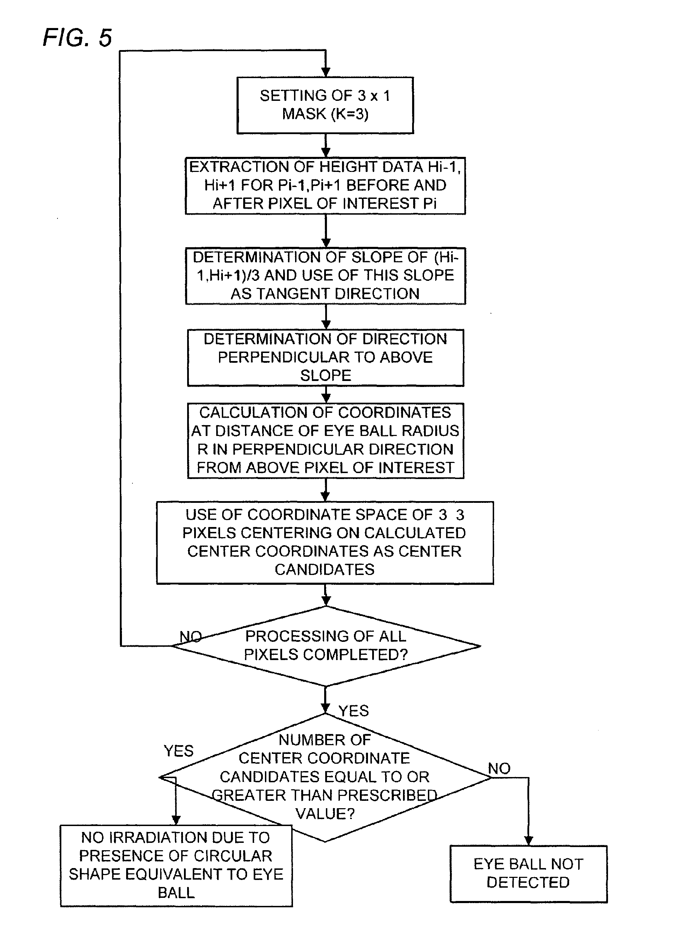

The following indicates an example of processing carried out by the irradiation determinator 5. In the case a curved shape possessed by an eye ball is taken to be the above-mentioned predetermined shape, a linear mask measuring K × 1 as shown in FIG. 3 is set. Since an eye ball has a spherical shape, a tangent is determined as shown in FIG. 4 from adjacent height data at each point for each of the K number of points of the mask, and a direction P that is perpendicular to the direction of the tangent is then determined. In the case of a circle, since a perpendicular direction determined from three points adjacent to a point of interest intersects the center of the circle, and the radius R thereof is equal to that of an eye ball, center position candidate coordinates from the direction P and radius R of each point. This processing is carried out based on the operation shown in the flow chart of FIG. 5, and once a certain minimum number of center position candidates have been calculated for the position considered to be the center position, a curved shape the same as that of an eye ball is judged to be present. At this time, the irradiation determinator 3 prevents emission of modulating light by prohibiting operation of the modulating light irradiator 10.

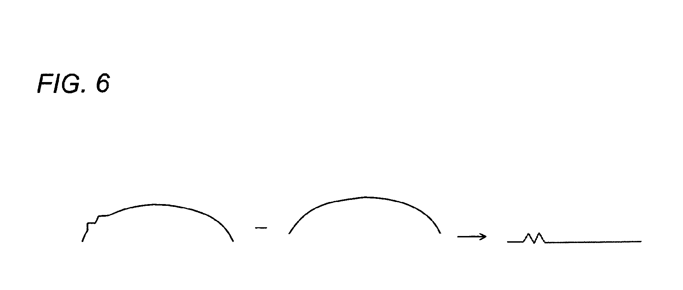

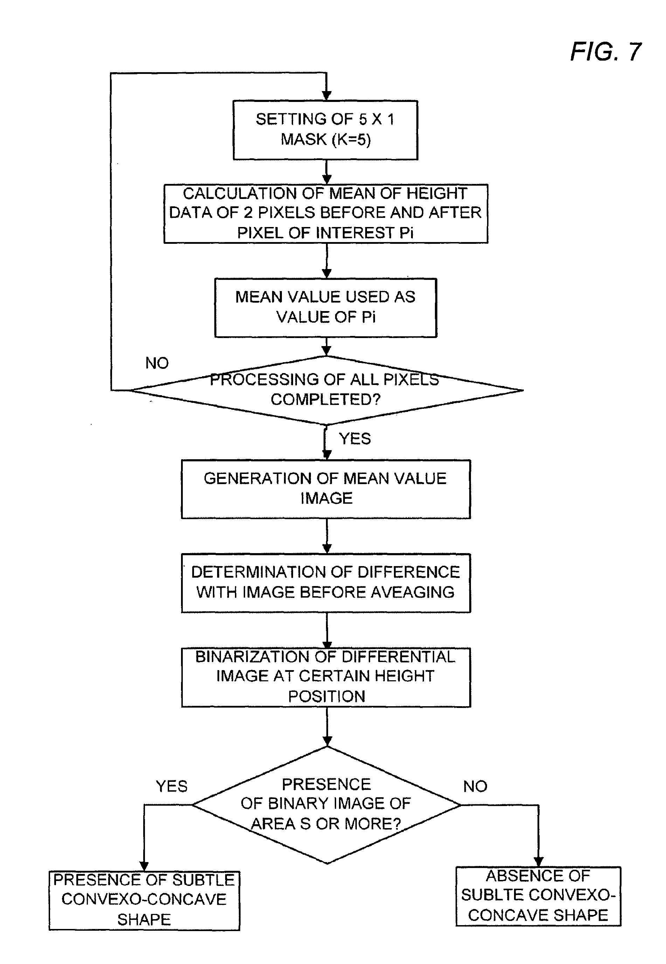

Moreover, in consideration of having to avoid irradiation of light in cases of the presence of projections on the skin caused by a rash, the device of the present invention is configured to prohibit irradiation of modulating light in such cases. In order to achieve this object, the irradiation determinator 50 generates data in which an M x M mean filter is applied to three-dimensional topographic data acquired from the data memory 44. This mean filter is for removing the curved shape of the skin, the value of M differs according to the site, and the value of M is set to increase in the case of a cheek and decrease more in the case of an arm or leg than in the case of a cheek. The irradiation determinator 50 generates images in which three-dimensional data to which the above-mentioned mean filter has been applied is subtracted from the original three-dimensional topographic data. In the case projections caused by a rash are present on the skin, corresponding subtle projections are included in the data as shown in FIG. 6. The irradiation determinator 50 further binarizes the three-dimensional data in which subtle projections are remaining using a certain value in the direction of height, and when sites having a height equal to or greater than a certain height are present at a prescribed value or more, the irradiator determinator 50 judges there to be subtle convexo-concave shapes present on the skin surface 9 and prohibits irradiation of modulating light from the modulating light irradiator 10. FIG. 7 shows a flow chart of this processing. Furthermore, since the value of K in the drawing is equivalent to the frequency of convexo-concave shapes, it can be determined from the size of the convexo-concave shapes and the measurement resolution.

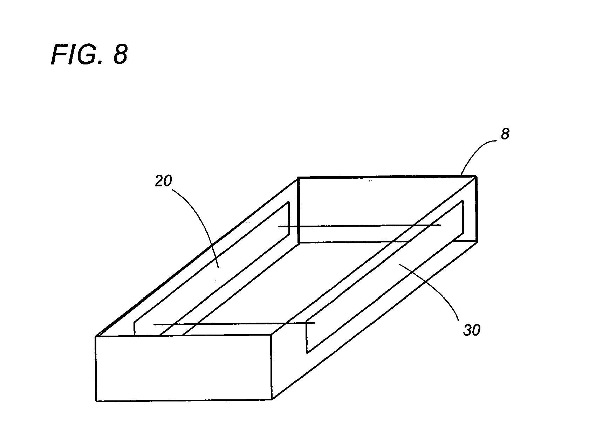

Since the presence or absence of certain hair around the periphery of an eye ball can also be confirmed by the same method as that described above, light is not irradiated in the case the hair is judged to be hair around the periphery of an eye ball. In the case of also recognizing the presence or absence of hair, this may be carried out by receiving reflected light with a line sensor by irradiating line light in a direction parallel to the curved shape of the skin instead of the measurement of three-dimensional shape as previously described, and in this case, the presence or absence of hair can be determined easily. However, since the curved shape of the skin can cause measurement error, by using a measuring head, in which the measuring light irradiator 20 and the light receiver 30 are arranged within a frame body 8 as shown in FIG. 8, and pressing the frame body 8 against the skin surface, the effects of the curved shape of the skin can be reduced.

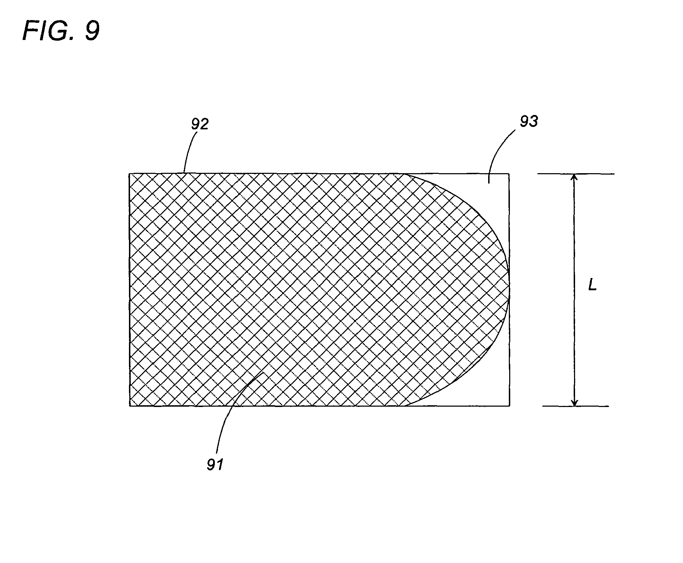

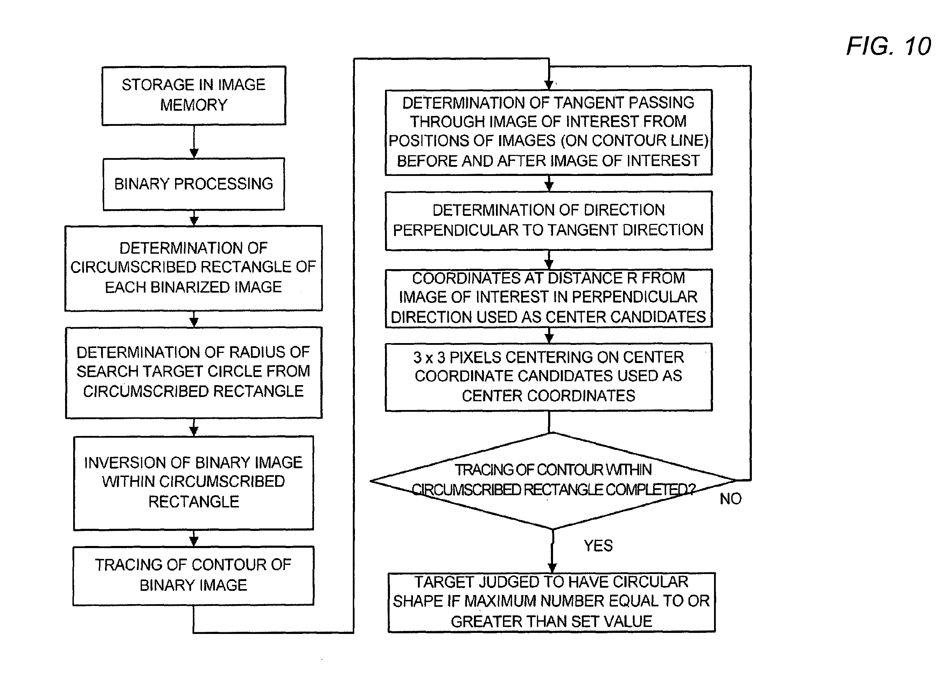

Moreover, with respect to moles as well, it is preferable to not irradiate moles with modulating light from the modulating light irradiator 10. In this case, the irradiation determinator 50 detects a mole using the procedure indicated in the flow chart of FIG. 10 and then prohibits irradiation of modulating light. Since moles are present on the skin surface in the form of round protrusions, in the case of irradiating measuring light from the measuring light irradiator 20 on an angle as previously described, a black shadow forms. Namely, by carrying out binarization on an image obtained with the light receiver 30 by assigning a value of 1 to portions for which brightness is equal to or less than a certain value, a shadow is extracted, a circumscribed rectangle 92 of a shadow 91 as indicated in FIG. 9 is then determined, and a judgment is made as to whether or not there is a circular shape within that range. The direction is recognized in which a region 93 is present that is not judged to have a value of 1 as a result of binarizing the range of the circumscribed rectangle 92, and in the case that direction is the long side of the circumscribed rectangle 92, the short side is presumed to be a circular shape having a circle diameter L, while in the opposite case, the long side is presumed to be a circular shape having a circle diameter L.

Next, the direction of the tangent of the circle is determined in the same manner as previously described from two pixels adjacent to a pixel of interest by tracing the contour of the binarized image, followed by determining the direction perpendicular to that tangent direction. Since the center of the circle is present in that perpendicular direction, voting is carried out on those points at a distance equal to the radius of the presumed circle in this perpendicular direction, and after repeating similar processing on all points on the contour of the binarized image, the coordinate having a certain number of votes or more is judged to be the center position of the circle, the binarized image is recognized to be the shadow of a circular object, and the irradiation determinator 50 then prohibits irradiation of modulating light.

Although the above-mentioned embodiment has explained the case of determining three-dimensional topographic data of the skin surface in the processor 40, the present invention is not necessarily limited thereto, but rather the processing 40 can be configured to determine a radius of curvature of a cross-section of a target skin surface using a light-section method. In this case, the irradiation determinator 50 is configured to judge not to irradiate the modulating light from the modulating light irradiator 10 in the case the determined curvature is the same as a preliminarily given radius curvature, such as the curvature of a human eye ball.

Furthermore, in the case of using polarized light for the measuring light irradiated from the measuring light irradiator 20, and receiving the measuring light that has reflected from the skin with the light receiver 30 after passing through the polarizing filter 32, the irradiation determinator 50 may also refer to a value of the amount of received light when judging whether or not a target site is an eye ball. Namely, although the direction of polarization of incident light has the property of rotating since tissue aligned in a fixed direction in the manner of muscle is present within skin tissue, since an eye ball is composed of the cornea, lens and the like, the direction of polarization of light appearing outside the eye after being reflected in the fundus does not change. Thus, if the angle of the polarizing filter 32 arranged in front of the light receiver 30 is taken to be a direction of 90° perpendicular to the direction of polarization of incident light, in the case the target site is skin, an amount of light can be obtained to a certain degree even if light is received in a direction in which the angle of polarization has rotated 90° due to the occurrence of polarization within the skin, while in contrast, in the case the target site is an eye ball, the amount of light received by the light receiver 30 is extremely small since polarization does not occur. By taking advantage of this phenomenon, the irradiation determinator 50 judges whether or not the target site is an eye ball. Namely, if the amount of measuring light received after passing through the polarizing filter 32 is equal to or less than a prescribed value, then the target site is judged to be an eye ball and the irradiation determinator 50 is configured to prohibit irradiation of modulating light.

In addition, since light irradiated by the measuring light irradiator 20 includes light having wavelengths of 1430 nm and 1940 nm that is absorbed by water, and an eye ball can be distinguished from skin by measuring absorbance of the reflected light, the irradiation determinator 50 can also use this phenomenon as a judgment criterion.

In relation thereto, in the case the irradiation determinator 50 carries out judgments using only the phenomenon of polarized light or optical absorbance, it can be configured in the form of a single-element light receiving means in the manner of using the measuring light irradiator 20 as a light source and using the light receiver 30 as a photodiode.

### (Second Embodiment)

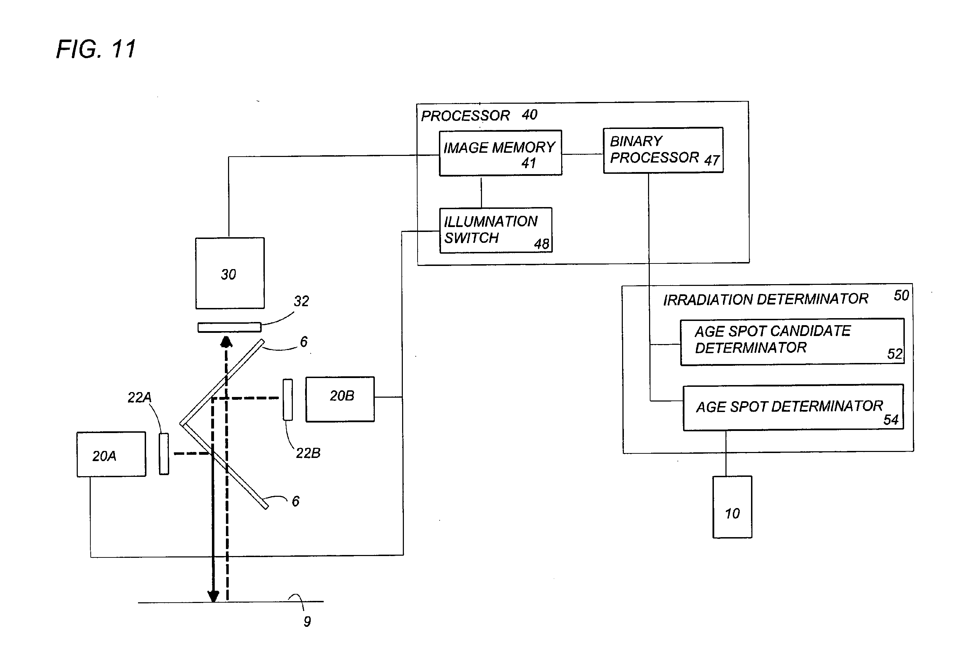

FIG. 11 shows a hair growth modulation device as claimed in a second embodiment of the present invention. This device is configured to irradiated modulating light for promoting hair removal effects by inhibiting hair growth, and is also configured to prohibit irradiation of modulating light at sites where age spots are present on the skin surface. In addition to being provided with the modulating light irradiator 10 in the same manner as the first embodiment, this device uses two measuring light irradiators 20A and 20B. Both of these measuring light irradiators are configured to irradiate measuring light having a wavelength of 200 to 400 nm, the first measuring light irradiator 20A uses an ultraviolet LED, while the second measuring light irradiator 20B uses a blue LED. The light receiver 30 is arranged so as to receive measuring light reflected from the skin surface, outputs electric signals corresponding thereto, and composes a coaxial reflecting optical system with each measuring light irradiator 20A and 20B and half mirrors 6, thereby preventing received measuring light from being affected by the texture of the skin surface 9. In addition, polarizing filters 22A, 22B and 32 are arranged in front of each of the measuring light irradiators 20A and 20B and the light receiver 30.

The processor 40 in this device is composed of the image memory 41 for storing images obtained from the light receiver 3, an illumination switch 48 for switching and illuminating the measuring light irradiators 20A and 20B, and a binary processor 47 for binarizing images. The irradiation determinator 50 for judging irradiation of modulating light from the modulating light irradiator 10 is composed of an age spot candidate determinator 52 and an age spot determinator 54.

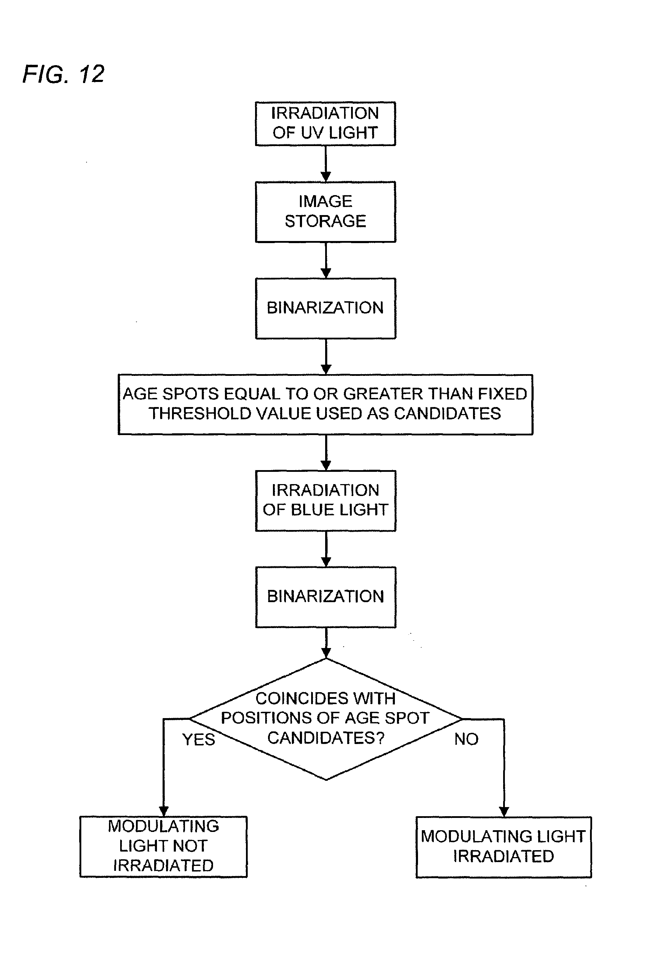

The following provides an explanation of the operation of this device based on the flow chart shown in FIG. 12. Prior to irradiation of light from the modulating light irradiator 10 to a target site to be irradiated, the processor 40 causes the measuring light irradiator 20A to turn on so that the measuring light irradiator 20A irradiates the ultraviolet measuring light to the target site. The light receiver 30 receives the light reflected by the skin surface 9. An output image of the light receiver 30 is then binarized by assigning a value of "1" to portions for which brightness is equal to or less than a certain value. The age spot candidate determinator 52 determines the total area of elements assigned a value of "1" according to this binary processing, and those portions for which this area is equal to or greater than a certain value are judged to be age spot candidates, while those portions for which this area is below that value are judged to be debris adhered to the skin.

Next, the processor 40 causes the measuring light irradiator 20b to turn on such that the measuring light irradiator 20b irradiates blue measuring light to the target site. The light receiver 30 receives the light reflected from the skin surface 9. At this time, light reflected from the skin surface is received by making the polarizing filter 22A arranged at the measuring light irradiator 20A and the polarizing filter 32 arranged at the light receiver 30 to have the same angle of polarization. On the other hand, the polarizing filter 22B arranged at the measuring light irradiator 20B and the polarizing filter 32 arranged at the light receiver 30 are made to have different angles of polarization by several degrees. Since light that has passed through the skin is affected by a change in the angle of polarization caused by internal tissue, a portion of the reflected light that has slightly entered the upper layer of the skin can be obtained with the light receiver 30. Since is because age spots are present not only on the skin surface but also in the upper layer of internal skin tissue, and an image of a portion of the age spot that has slightly entered the skin tissue is required to make an accurate judgment thereof. As a result of employing this procedure, light received by the light receiver 30 is resistant to the effects of the skin surface, thereby reducing the probability of incorrectly recognizing dirt or debris adhered to the skin surface.

Images obtained in this manner are subjected to binary processing in the same manner as described above in the binary processing 47. The age spot determinator 54 then extracts a portion for which the total area of pixels assigned a value of "1" by the binary processing is equal to or greater than a certain value, and if that portion coincides with a portion determined to be an age spot candidate as previously described, that portion is judged to be an age spot and irradiation of modulating light from the modulating light irradiator 10 is prohibited, while if it does not coincide, irradiation of modulating light from the modulating light irradiator 10 is permitted.

The use of ultraviolet light and blue light having a wavelength of 200 to 400 nm for the measuring light is effective for judging age spots. This is because in comparison with light having a longer wavelength, this light has difficulty in entering the skin resulting in greater reflectance at the surface.

In the case of using polarizing light for the measuring light and receiving light with the light receiver 30 after passing through the polarizing filter 32, a judgment can also be made as to whether or not a target site is an eye ball in the same manner as the previously described embodiment.

In addition, if the wavelength of the measuring light from the measuring light irradiator 20 includes light having a wavelength of 1430 nm and 1940 nm that is absorbed by water, an eye ball can be distinguished from skin by measuring absorbance of the reflected light.

Although the present embodiment has indicated irradiation of modulating light of a wavelength that inhibits hair growth, it can also be configured to irradiate modulating light of a wavelength that promotes hair growth.

### (Third Embodiment)

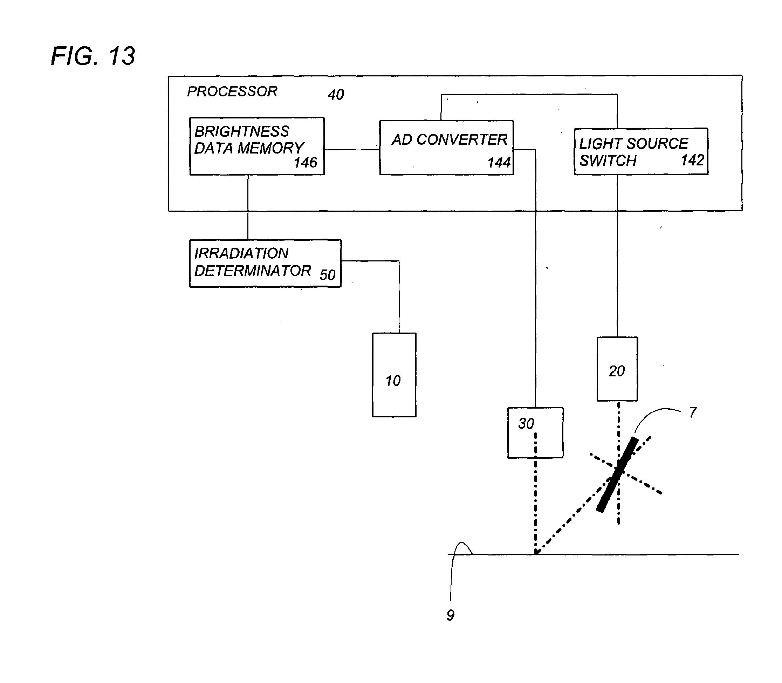

FIG. 13 shows a hair growth modulation device as claimed in a third embodiment of the present invention. This device is configured to discontinue irradiation of modulating light when a target site has been judged to be an eye ball, and similar to the first embodiment, is provided with a modulating light irradiator 10 for irradiating modulating light, a measuring light irradiator 20 for irradiating measuring light, and a light receiver 30 for receiving measuring light reflected from a skin surface, while also being further provided with the processor 40 for processing electric signals output from the light receiver 30, and the irradiation determinator 50 for judging whether irradiation of modulating light is to be permitted based on data processed by the processor 40.

The measuring light irradiator 20 has an LED that irradiates three types of red, green and blue (RGB) measuring light, and is arranged so as to irradiate measuring light at an angle of 45° to the skin surface 9 with a reflection mirror 7, while a light switch 142 switches the RGB optical output at prescribed intervals. The light receiver 30 is composed of a photodiode, and light that has been received thereby is accumulated in brightness data memory 146 for each RGB after respectively passing through an A/D converter 144.

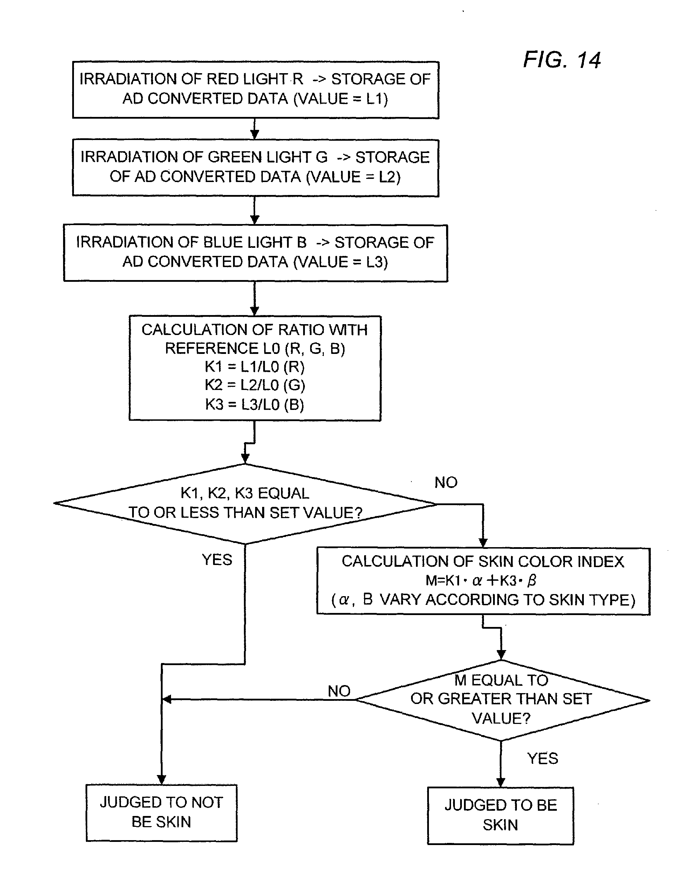

Although the skin surface has the color of skin due to the effects of capillaries and melanin, the eye ball itself has few blood vessels and appears black due to the high proportion of melanin. Consequently, when irradiated with three color lights of RGB, in the case the target is black, only a phenomenon in which reflectance decreases occurs, and the amount of reflected light for each of the RGB lights decreases at about the same rate. On the other hand, in the case of skin, the rate of decrease in reflectance is not constant. In the device as claimed in the present embodiment, in the case all of ratios with the reference value L0 (K1 = L1/L0, K2 = L2/L0, K3 = L3/L0) are equal to or less than a set value for the brightness of each of the colors red, green and blue (L1, L2, L3) as shown in the flow chart of FIG. 14, the target site is judged to be a site other than skin (eye ball). When all of the ratios are not equal to or less than the set value, a skin color index (M = K1·α + K3·β) is calculated, and in the case that skin color index M is equal to or greater than a prescribed value, the target site is judged to be skin and irradiation of modulating light is permitted. In the case the skin color index M is less than the prescribed value, the target site is judged to not be skin and irradiation of modulating light is prohibited. Values suitably determined according to the skin type of a user are used for the values of α and β.

### (Fourth Embodiment)

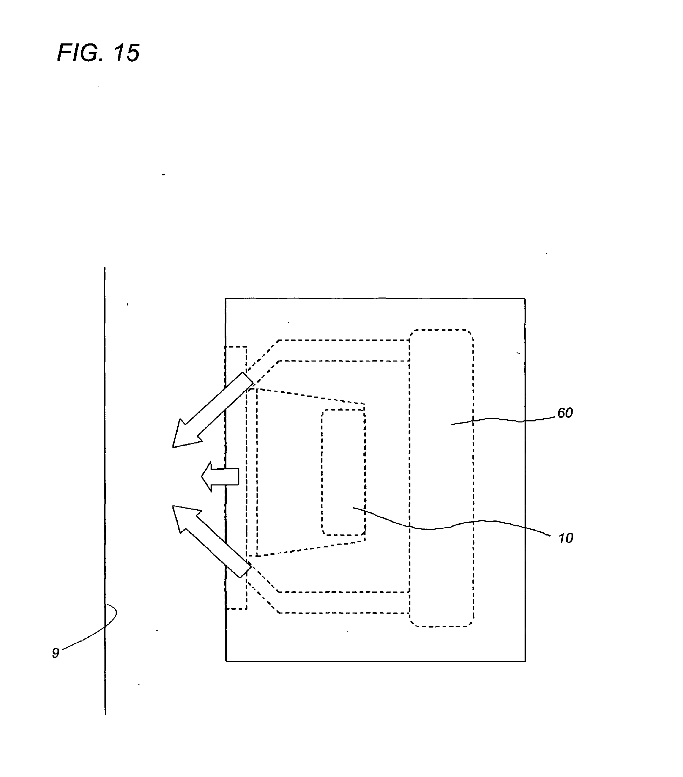

FIG. 15 shows a hair growth modulation device as claimed in a fourth embodiment of the present invention. Although this device is basically similar to that of the first embodiment, it is provided with a weak stimulus generator 60 that imparts a weak stimulus to an eye of a user before irradiating with modulating light. In this device, by imparting this weak stimulus so as to cause the eye of the user to close and irradiating skin with the modulating light during that time, it is intended for the device to be able to be used without any discomfort, and this is particularly effective in the case of irradiating the modulating light in close proximity to the eye.

The following provides an explanation of the weak stimulus generator 60 in the devices as claimed in the present embodiment. The weak stimulus generator 60 imparts a weak stimulus to a target site 9 prior to irradiating the modulating light from the modulating light irradiator 10. As a result of this weak stimulus, the eye is closed and during the time the eye is closed, the modulating light is irradiated from the modulating light irradiator 10. Although the weak stimulus generator 60 may continue to impart the weak stimulus to cause the eye to close, imparting the weak stimulus only for a time that is adequate for causing the eye to close makes it possible to prevent waste of excess energy. The time until the modulating light is irradiated after the weak stimulus generator 60 imparts the weak stimulus to the target site is set in consideration of the time from imparting the weak stimulus to the eye until the eye closes and the time the eye is caused to close by the weak stimulus.

Although spraying of a gas having a cooling effect is preferable for the weak stimulus, the weak stimulus is not limited to spraying of a gas, but may also be in the form of light or an electric current.

Spraying of gas can be carried out by, for example, providing a known spraying mechanism in the weak stimulus generator 60. In addition, wind pressure can also be generated by providing a fan, for example, in the weak stimulus generator 60. The spraying of gas is required to cause the eye to close while also being safe for the eye. Furthermore, although there is the risk of burns if the skin is excessively irradiated with the modulating light from the modulating light irradiator 10, the use of gas having a cooling effect makes it possible to reduce this risk.

In the case of imparting a weak stimulus with light, a light irradiator is provided in the weak stimulus generator 60. This irradiation of light is required to cause the eye to close while also being safe for the eye. Furthermore, since humans are more easily responsive to changing light rather than fixed light, the light that causes the eye to close is preferably, for example, pulsed light or flashing light. In addition, since the wavelength of light having high visual sensitivity that causes the human eye to blink due to a physiological response at the lowest possible power (for example, at the LED level) is 555 nm, it is preferable to impart the weak stimulus with light of this wavelength.

In the case of using electric current, a current generator is provided in the weak stimulus generator 60. The current used is weak and is made to flow around the periphery of the eye. This current is required to cause the eye to close while also being safe to the eye.

Furthermore, although the aforementioned description has indicated three functions of the weak stimulus 2, these may be arbitrarily combined to allow a plurality of functions to coexist.

Thus, since the weak stimulus 2 imparts a weak stimulus to the eye that causes the eye to close, and light that modulates growth of body hair is irradiated by the light irradiator 1 A during the time the eye is closed due to the weak stimulus, the eye can be protected from the light that modulates growth of body hair.

### (Fifth Embodiment)

FIG. 15 indicates a hair growth modulation device as claimed in a fourth embodiment of the present invention. Although this device is similar to that of the fourth embodiment, it differs from the above-mentioned embodiment in that the modulating light irradiator 10 is also used as the weak stimulus generator 60. The modulating light irradiator 10 in this embodiment is configured to irradiate weak light that does not impart damage to the eye ball prior to irradiating modulating light. Furthermore, a weak stimulus generator that imparts a weak stimulus other than light can also be additionally provided.

## Claims

1. A hair growth modulation device comprising:
a modulating light irradiator configured to irradiate a modulating light for modulation of hair growth to a target site of a human body;
a measuring light irradiator configured to irradiate a measuring light for measurement of the human body to said target site of the human body;
a light receiver configured to receive the measuring light reflected from said target site;
a processor configured to process information of the measuring light received at said light receiver; and
an irradiation determinator which makes a judgment of whether or not to make the light irradiation from said modulating light irradiator based upon a
processing result at said processor,
wherein said irradiation determinator is configured to control said modulating light from said modulating light irradiator in accordance with said judgment.

2. A hair growth modulation device as set forth in claim 1, wherein said processor is configured to output an estimated shape of the target site, said irradiation determinator is configured to make the judgment of not irradiating the modulating light from said modulating light irradiator when said estimated shape coincides with a human eye ball shape.

3. A hair growth modulation device as set forth in claim 1, wherein said processor is configured to output an estimated shape of the target site, said irradiation determinator is configured to make the judgment of not irradiating the modulating light from said modulating light irradiator when said estimated shape includes a regularly repeated pattern of convexo-concave shape.

4. A hair growth modulation device as set forth in claim 1, wherein said processor is configured to obtain curvature of a cross-section at said target site by means of a light-section method and output the curvature, said irradiation determinator is configured to make the judgment of not irradiating the modulating light from said modulating light irradiator when the curvature coincides with the curvature inherent to the human eye ball.

5. A hair growth modulation device as set forth in claim 1, wherein said measuring light irradiator is configured to irradiate the measuring light which is polarized in a particular direction, said light receiver is configured to obtain polarization information about the measuring light.

6. A hair growth modulation device as set forth in claim 1, wherein said processor is configured to calculate the amount of the light received at said light receiver,
said irradiation determinator is configured to make the judgment of whether or not to make the light irradiation from said modulating light irradiator based upon the amount of the measuring light.

7. A hair growth modulation device as set forth in claim 6, wherein said measuring light irradiator is configured to irradiate said measuring light having a wavelength in a range of 200 nm to 400 nm.

8. A hair growth modulation device as set forth in claim 6, wherein
said light receiver has a plurality of light receiving elements arranged in a two-dimensional array,
said irradiation determinator is configured to make the judgment of not making the modulating light irradiation from said modulating light irradiator when the number of the light receiving elements each seeing the amount of the received light below a predetermined level exceeds a predetermined number.

9. A hair growth modulation device as set forth in claim 6, wherein
said measuring light irradiator is configured to irradiate the measuring light polarized in a particular direction, and
said light receiver is configured to obtain polarized information of the received light.

10. A hair growth modulation device as set forth in any one of claims 7 to 9,
wherein
said measuring light irradiator is configured to irradiate the three color measuring lights of RGB, respectively, and
said light receiver is configured to measure the amount of each color of RGB.
